Europäisches Patentamt

European Patent Office

Office européen des brevets

(19)

(11) **EP 1 212 448 B1**

(12) **FASCICULE DE BREVET EUROPEEN**

(45) Date de publication et mention
de la délivrance du brevet:
**12.11.2003 Bulletin 2003/46**

(51) Int Cl.⁷: **C12P 19/04**, C12P 19/06,
C12N 1/14, C12N 1/20,
C08B 37/00

(21) Numéro de dépôt: **00960805.0**

(22) Date de dépôt: **06.09.2000**

(86) Numéro de dépôt international:
**PCT/FR00/02452**

(87) Numéro de publication internationale:
**WO 01/018226 (15.03.2001 Gazette 2001/11)**

(54) **PROCEDE DE PRODUCTION D'EXOPOLYSACCHARIDES**

VERFAHREN ZUR HERSTELLUNG VON EXOPOLYSACCHARIDE

METHOD FOR PRODUCING EXOPOLYSACCHARIDES

(84) Etats contractants désignés:
**AT BE CH CY DE DK ES FI FR GB GR IE IT LI LU
MC NL PT SE**

(30) Priorité: **07.09.1999 FR 9911176**

(43) Date de publication de la demande:
**12.06.2002 Bulletin 2002/24**

(73) Titulaire: **RHODIA CHIMIE**
**92512 Boulogne Billancourt Cedex (FR)**

(72) Inventeurs:
• **NORE, Olivier**
**37270 AZAY-SUR-CHER (FR)**
• **SIMON, Jean-Luc**
**59000 LILLE (FR)**

(74) Mandataire: **Dubruc, Philippe et al**
**Rhodia Services,**
**Direction de la Propriété Industrielle,**
**40, rue de la Haie-Coq**
**93306 Aubervilliers Cedex (FR)**

(56) Documents cités:
• **ROUKAS TRIANTAFYLLOS ET AL: "Evaluation
of carob pod as a substrate for pullulan
production by Aureobasidium pullulans."
APPLIED BIOCHEMISTRY AND
BIOTECHNOLOGY, vol. 55, no. 1, octobre 1995
(1995-10), pages 27-44, XP000915013 ISSN:
0273-2289**
• **ROSEIRO J C ET AL: "BATCH AND FED-BATCH
CULTIVATION OF
XANTHOMONAS-CAMPESTRIS IN CAROB
EXTRACTS" LEBENSMITTEL-WISSENSCHAFT
& TECHNOLOGIE, vol. 25, no. 3, 1992, pages
289-293, XP000922972 ISSN: 0023-6438**

**Description**

La présente invention a pour objet un procédé pour la production d'exopolysaccharides par fermentation au moyen de microorganismes. Plus spécifiquement, l'invention concerne un procédé pour la production d'exopolysaccharides par fermentation de microorganismes dans un milieu nutritif comportant au moins une source de carbone assimilable par les microorganismes et au moins une source organique d'azote dérivant d'une légumineuse possédant une teneur élévée en protéines.

Dans le cadre de la présente invention, le terme exopolysaccharide désigne les polysaccharides produits par des microorganismes.

Les exopolysaccharides de poids moléculaire élevé sont utilisés de manière croissante dans de nombreuses applications industrielles pour leurs propriétés épaississantes, viscosifiantes, émulsifiantes, stabilisantes dans les milieux notamment aqueux. Ainsi, la gomme xanthane, en raison de ses propriétés rhéologiques exceptionnelles, est utilisée dans des domaines aussi variés que le bâtiment, la peinture, le papier, le textile, les cosmétiques, l'alimentaire, l'agriculture, le traitement des eaux, le forage, la récupération du pétrole et autres.

Ces exopolysaccharides ont des poids moléculaires élevés, le plus souvent supérieurs à $1.10^6$ g/mol (mesurés par perméation de gel), et sont constitués d'unités de glucose, mannose, galactose, rhamnose, acide glucuronique, acide mannuronique, acide guluronique, avec éventuellement des dérivés acétate et pyruvate. Leur structure particulière et leurs propriétés sont décrites par exemple dans l'ouvrage Industrial Gums - Whistler - 2nd Edition - Chapters XXI-XXIII (1973).

Les exopolysaccharides sont produits avantageusement par culture aérobie de microorganismes dans un milieu nutritif aqueux.

La gomme xanthane est produite par des bactéries du genre *Xanthomonas*. Les exopolysaccharides du même type peuvent être produits par une grande variété de microorganismes incluant parmi les plus notoires, ceux du genre *Agrobacterium, Arthrobacter, Alcaligenes* (Succinoglycane), *Pseudomonas* (Levan), *Rhizobium, Sclerotium* (Scléroglucane).

Le milieu nutritif aqueux comprend normalement, outre des éléments de croissance variés, une source de carbone et une source d'azote. Dans les fermentations industrielles, le choix de la source de carbone et/ou de la source d'azote est basé à la fois sur sa disponibilité, sur son coût et sur son aptitude à permettre des productivités élevées.

Notamment il est connu par le document « Evaluation of carob pod as a substrate for Pullulan production by Aureobasidium pullulans »; Triantafyllos R. et al. Applied Biochemistry and Biotechnology, vol. 55, N°1, 1995 p.27-44 un procédé de production de pullulons dans un milieu nutritif dérivant d'un extrait de caroube riche en sucres provenant de la cosse du fruit. Il est également connu par « Batch and fed-batch cultivation of Xanthamonas campestris in carob extracts » Roseiro J.C. et al. Lebensmittel-Wissenschaft & Technologie, vol. 25, N°3, 1992 p.289-293 un procédé de production de gomme xanthane dans un milieu nutritif dérivant d'un extrait de caroube riche en sucres provenant de la cosse du fruit.

Dans certaines industries comme par exemple l'industrie alimentaire ou cosmétique, des supplémentaires interviennent. Dans ces domaines, les sources de carbone et d'azote doivent, en outre, être choisies de manière à obtenir des exopolysaccharides satisfaisant aux exigences organoloptiques, sensorielles, et visuelles recherchées.

Parmi les sources de carbone et d'azote habituellement utilisées, il n'est pas facile de trouver des sources qui répondent à la fois à toutes les exigences précitées.

Par exemple, dans les cas où le microorganisme n'est pas capable de consommer la totalité de la source d'azote, il reste des produits résiduels insolubles en fin de fermentation qui d'une part rendent le milieu propice au développement de souches contaminantes pouvant dégrader le moût avant séparation de l'exopolysaccharide, et d'autre part risquent de colorer l'exopolysaccharide lors des traitements thermiques de stérilisation et de clarification éventuels. Dans certains procédés de fermentation, pour remédier à cet inconvénient on propose d'utiliser des enzymes. D'autres mettent en oeuvre des étapes de filtration et/ou de centrifugation. Quel que soit le procédé d'élimination de produits résiduels insolubles de fin de fermentation, il en résulte un coût de production accru.

Certaines sources de carbone et/ou d'azote présentent l'inconvénient d'allonger considérablement le cycle de fermentation entraînant notamment la contamination et donc la dégradation du moût avant séparation de l'exopolysaccharide, et la perte de production.

La nature de la source d'azote est particulièrement importante lorsque l'on cherche à obtenir un exopolysaccharide présentant de bonnes propriétés organoloptiques, sensorielles et visuelles. Elle est aussi responsable de la bonne productivité de l'exopolysaccharide.

Il a été constaté que certaines sources dérivant d'une fraction de la graine de certaines légumineuses comme la caroube, était une source d'azote organique particulièrement intéressante dans la fermentation des microorganismes. Ces fractions se sont avérées satisfaire à l'ensemble des exigences précitées.

Parmi les fractions dérivant de la graine de caroube, celles possédant avantageusement une teneur élevée en protéines donnent des résultats particulièrement intéréssants notamment en terme de productivité. Sur des milieux dit standards tels que ceux mentionnés par exemple dans l'ouvrage Industrial Gums - Whistler-

2nd Edition - Chapters XXI-XXIII (1973), pour des fermentations témoin, les productivités sont de l'ordre de 0,3 à 0,4 g/(kg.h) ; pour les fractions dérivant de la graine de caroube cette productivité est supérieure à 0,4 g/(kg.h).

La présente invention a pour but de proposer un procédé de production d'exopolysaccharides par fermentation de microorganismes qui soit simple et économique.

Un autre but de l'invention est de proposer un procédé de production d'exopolysaccharides par fermentation de microorganismes qui évite les problèmes de contamination exposés ci-dessus.

Ainsi, l'invention a pour objet un procédé de production d'exopolysaccharides par fermentation de microorganismes caractérisé en ce que l'on conduit la fermentation dans un milieu nutritif comportant au moins une source de carbone assimilable par les microorganismes et au moins une source organique d'azote, ladite source dérivant d'une fraction de la graine de caroube.

D'autres avantages liés au choix notamment de la source d'azote sont la diminution de la durée des fermentations, la suppression de produits résiduels insolubles de fin de fermentation et une productivité améliorée.

De plus, ce procédé permet d'obtenir un exopolysaccharide ayant de bonnes propriétés organoleptiques, sensorielles et visuelles.

Par ailleurs, les propriétés rhéologiques de l'exopolysaccharide obtenu par ce procédé sont préservées et même dans certains cas améliorées.

Le procédé de l'invention est susceptible d'être appliqué à la production de tout exopolysaccharide par fermentation au moyen de microorganismes. De nombreux microorganismes tels que bactéries, levures, champignons, algues, sont capables de produire des exopolysaccharides. On peut citer entre autres :

■ des bactéries appartenant au genre *Xanthomonas* et plus particulièrement aux espèces décrites dans Bergey's Manual of Determinative Bacteriology (8ᵉ édition - 1974 - Williams N. Wilkins Co. Baltimore) telles que *Xanthomonas begoniae, Xanthomonas campestris, Xanthomonas carotae, Xanthomonas hederae, Xanthomonas incanae, Xanthomonas malvacearum, Xanthomonas papavericola, Xanthomonas phaseoli, Xanthomonas pisi, Xanthomonas vasculorum, Xanthomonas vesicatoria, Xanthomonas vitians, Xanthomonas pelargonii ;*
■ des bactéries appartenant au genre *Arthrobacter* et plus particulièrement les espèces *Arthrobacter stabilis, Arthrobacter viscosus ;*
■ des bactéries appartenant au genre *Erwinina ;*
■ des bactéries appartenant au genre *Azotobacter* et plus particulièrement l'espèce *Azotobacter indicus ;*
■ des bactéries au genre *Agrobacterium* et plus particulièrement les espèces *Agrobacterium radiobacter, Agrobacterium rhizogenes, Agrobacterium tumefaciens ;*
■ des bactéries appartenant au genre *Alcaligenes* et plus particulièrement *Alcaligenes faecalis ;*
■ des bactéries appatenant au genre *Pseudomonas* et plus particulièrement *Pseudomonas methanica ;*
■ des bactéries appartenant au genre *Corynebacterium ;*
■ des bactéries appartenant au genre *Bacillus* et plus particulièrement *Bacillus polymyxa;*
■ des champignons appartenant au genre *Sclerotium* et plus particulièrement aux espèces *Sclerotium glucanicum, Sclerotium rolfsii* ou *Plectania occidentalis ;*
■ des champignons appartenant au genre *Aspergillus* et plus particulièrement aux espèces *Aspergillus itaconicus, Aspergillus terreus :*
■ des levures appartenant au genre *Hansenula* comme l'espèce *Hansenula capsulata.*

De préférence le microorganisme est une bactérie du genre *Xanthomonas* et plus particulièrement de l'espèce *Xanthomonas campestris.*

L'invention a pour objet principal un procédé de production d'exopolysaccharides par fermentation de microorganismes caractérisé en ce que l'on conduit la fermentation dans un milieu nutritif comportant au moins une source de carbone assimilable par les microorganismes et au moins une source organique d'azote, ladite source étant dérivée d'une fraction de la graine de caroube.

L'arbre de caroubier produit un fruit, constitué de deux parties, la cosse et la graine. La graine de caroube, et plus particulièrement la fraction endosperme de cette graine, est déjà largement valorisée sous la dénomination " gomme de caroube". Contigu à cette fraction endosperme, se trouve le germe qui est un sous produit obtenu en quantités importantes lors de l'isolement de la gomme de caroube.

Parmi les différentes fractions de la graine de caroube, il s'est avéré que toutes celles ayant une teneur élevée en protéines étaient plus particulièrement adaptées au procédé de la présente invention.

Ainsi, la fraction de la graine de caroube possède avantageusement une teneur en protéines d'au moins 45 %, de préférence d'au moins 50 %, et plus préférentiellement d'au moins 60 %, en poids par rapport au poids sec de la matière sèche.

La teneur en protéine est calculée à partir de la mesure de l'azote libéré par combustion à 950°C sous oxygène et mesuré par conductivité dans un flux d'hélium. L'appareillage utilisé est un LECO FP 428.

Ces protéines sont constituées aussi bien des acides aminés essentiels que des acides aminés non essentiels.

Un mode de réalisation particulier de l'invention consiste à mettre en oeuvre des fractions de la graines de caroube dont les protéines ont avantageusement un taux élevé en arginine, en glutamine et/ou en acide glutamique, et en lysine.

Dans ce mode particulier, la teneur en arginine est avantageusement comprise entre 9 et 20 %, et de préférence entre 12 et 14 %, poids/poids par rapport à l'ensemble des acides aminés.

De la même façon, la teneur en glutamine et/ou acide glutamique est avantageusement comprise entre 18 et 30 %, de préférence entre 22 et 27%, poids/poids par rapport à l'ensemble des acides aminés.

La teneur en lysine est avantageusement comprise entre 18 et 30 %, de préférence entre 12 et 14 %, poids/poids par rapport à l'ensemble des acides aminés.

La teneur en acides aminés est déterminée par des méthodes classiques et connues de l'homme du métier.

Outre les protéines, les fractions peuvent également comporter des lipides. Les exopolysaccharides produits par fermentation de microorganismes dans un milieu nutritif comportant au moins une source organique d'azote dérivée d'une fraction de la graine de caroube comportant des lipides, voient plus particulièrement leurs propriétés organoloptiques, visuelles et sensorielles sensiblement améliorées. Ces lipides empéchent également le moussage dans les phases de préculture.

Avantageusement, la teneur en lipides dans lesdites fractions est d'au moins 4%, avantageusement d'au moins 5 %, et encore plus avantageusement varie entre 7 et 15% en poids par rapport à la matière sèche.

La teneur en lipides est ramenée à celle de la matière grasse totale. Elle est déterminée par extraction à l'hexane dans un extracteur Soxhlet. Le mode opératoire est le suivant :

- on pèse dans la cartouche de l'extracteur, environ exactement 10 g de farine de germe de caroube, soit E grammes, obturés avec un tampon de coton hydrophile ;
- on introduit dans un ballon de 250 ml, préalablement taré (P0 gramme), 150 ml d'hexane ;
- on extrait pendant environ 6 heures ;
- on évapore le solvant à l'aide d'un évaporateur rotatif et on termine le séchage du résidu dans une étuve à 105°C pendant 1 heure ;
- après refroidissement dans un déssicateur, on pèse le ballon contenant le résidu, soit P1 grammes.

La teneur en matière grasse et donc en lipides est déterminée selon la formule suivante:

$$\text{Teneur en matière grasse (\%)} = 100 \times (P1 - P0) / E$$

Parmi les composants caractéristiques présents dans ces lipides, on peut citer notamment les acides palmitique, stéarique, oléique et linoléique.

Outre les protéines et les lipides, les fractions de la graine de caroube peuvent aussi comporter des hydrates de carbone.

Selon un mode de réalisation particulier de l'invention, ladite fraction est le germe de la graine de caroube.

Dans ce mode de réalisation, ladite fraction est préalablement débarassée de ses fractions endospermes selon les méthodes classiques connues.

La fraction de la graine de caroube utilisée peut être de préférence sous forme d'une farine. La farine est obtenue par des moyens classiques de broyage tels que broyage dans des moulins du type :

- moulins à cylindre pour les farines de granulométrie moyenne type mesh 100, c'est-à-dire une farine présentant au plus 1 % en masse de particules supérieures à 80 mesh et au plus 10 % en masse de particules inférieures à 200 mesh ;
- moulins à broches (pin mills) pour les farines de granulométrie plus fine :

  □ type mesh 200, c'est-à-dire une farine ne présentant pas de particules supérieures à 80 mesh et présentant au plus 60 % en masse de particules inférieures à 200 mesh, et
  □ type mesh 175, c'est-à-dire une farine présentant au plus 1 % en masse de particules supérieures à 80 mesh et au plus 75 % en masse de particules inférieures à 200 mesh.

La farine peut être utilisée telle quelle ou après traitement par des enzymes adaptées comme par exemple des protéases alcalines, acides et/ou neutres ; des lipases; des phytases; des phosphatases alcalines, acides et/ou neutres; des amylases. Le traitement par les enzymes se fait par des méthodes classiques et connues.

La granulométrie de ladite farine peut fluctuer entre 10 et 150 microns. Dans le cas des farines traitées, cette granulométrie est plus particulièrement de 20 à 60 microns, de préférence d 30 à 50 microns.

Les mesures de granulométrie peuvent être réalisées par la technique de granulométrie laser, à l'aide d'un granulomètre MALVERN, commercialisé par la société Malvern Instruments S.A.

On peut également envisager d'utiliser la fraction de la graine de caroube telle quelle, c'est-à-dire après séparation de l'endosperme sous forme de plaquettes,ou encore sous forme d'une pré-dispersion ou pré-suspension aqueuse.

Bien que l'invention soit décrite pour la graine de caroube, elle peut également s'appliquer à d'autres légumineuses comme par exemple le guar, le cassia, le tara. Ces légumineuses sont citées à titre indicatif et non limitatif.

Dans un autre mode de réalisation particulier de l'invention, la fermentation a lieu avec un mélange de sources d'azote organiques et minérales.

Dans ce cas, la source d'azote minérale peut être choisie parmi les nitrates d'ammonium ou de sodium, les phosphates ou les sulfates d'ammonium, sulfate de magnésium, sulfate de potassium ou de sodium, seuls ou en mélange.

La concentration en sources organiques et éventuellement minérales d'azote dans le milieu de fermentation est comprise entre 1 et 80 g/l, de préférence entre 3 et 50 g/l, et plus préférentiellement entre 5 et 30 g/l.

Le milieu de fermentation renferme également une source de carbone assimilable par les microorganismes.

A titre de source de carbone constitutive du milieu de fermentation, on peut citer le glucose, le saccharose, le fructose, le galactose, le tréhalose, le mannose, le mélobiose, le raffinose, le maltotriose, le maltose, le lactose, le lactulose, le méthyl-β-galactopyranoside, le méthyl-α-galactopyranoside, le cellobiose, le gentobiose, le mèthyl-β-D-glucopyranoside, le méthyl-α-D-glucopyranoside, l'esculine, le ribose, l'arabinose, le xylose, le palatinose, le rhamnose, le fucose, le mélézitose, le D(+) arabitol, le L(-) arabitol, le xylitol, le dulcitol, le tagatose, le glycérol, le myo-innositol, le mannitol, le maltitol, le turanose, le sorbitol, l'adonitol, le lyxose, l'érythritol, l'amidon avantageusement hydrolysé, les hydrolysats d'amidon, les mélanges de ces sucres, et les mélanges comprenant au moins un de ces sucres. Le glucose et le saccharose sont les sucres préférés.

La concentration en source de carbone assimilable est comprise entre 1 et 100 g/l, et de préférence entre 15 et 80 g/l.

Le milieu de fermentation peut, en outre, renfermer des oligo-éléments tels que des traces de sels minéraux tels que sulfates, chlorures de fer, de calcium, de manganèse, de magnésium, de sodium, de potassium, de nickel, de cobalt, de cuivre, de zinc ou leur mélange, ainsi que des vitamines, des nucléotides et/ou d'autres additifs conventionnels tels que des agents de contrôle de pH et des agents anti-moussants.

Le procédé de production d'exopolysaccharides selon l'invention par fermentation de microorganismes peut éventuellement se faire en présence d'enzyme(s) tels que des protéases alcalines, acides et/ou neutres ; des polysaccharases ; des amidases ; des peptidases , amyloglucosidases, phosphatases ; phytases.

Cependant, un des avantages importants du procédé selon l'invention réside dans le fait que l'on peut conduire la fermentation des microorganismes en l'absence d'enzyme. Il a été constaté de manière tout à fait surprenant qu'en l'absence d'enzyme, ni la durée ni la productivité du procédé de fermentation n'étaient affectées. De plus, la suppression d'enzyme n'a pas entraîné une accumulation des produits résiduels insolubles et non dissous en fin de fermentation qui peuvent rendre le milieu propice au développement de souches contaminantes pouvant dégrader le moût avant séparation de l'exopolysaccharide.

La culture pure des microorganismes peut être effectuée de manière classique. L'homme du métier, en fonction du microorganisme sera en mesure de choisir les conditions notamment les températures et temps d'incubation, et la nature du milieu d'entretien dudit microorganisme.

Pour la conservation du microorganisme, il est préférable de prévoir au moins une étape de préculture. Par préculture, on entend une étape qui consiste à développer et à multiplier la souche bactérienne, sans production d'exopolysaccharide.

Le microorganisme est introduit dans le milieu de fermentation de manière connue en soi à l'aide d'inoculums ou de cultures intermédiaires.

La fermentation peut être réalisée à des pressions comprises entre 0 et 4 bar.

On peut conduire la fermentation à une température comprise entre 15 et 100°C, de préférence entre 25 et 80°C, et plus particulièrement entre 25 et 35°C.

Le pH du milieu de fermentation peut varier entre 5 et 9, et de préférence entre 6 et 8. Le pH peut être ajusté, selon le cas, avec une base telle que la soude, la potasse, ou l'ammoniaque, ou avec un acide tel que l'acide sulfurique, l'acide phosphorique, l'acide chlorhydrique ou l'acide nitrique.

Le milieu de fermentation, placé dans une cuve ou un récipient de fermentation, peut être avantageusement soumis à une agitation et à une aération. Cette agitation peut être exercée par exemple au moyen d'un secoueur réciproque, d'un secoueur giratoire, d'un ou plusieurs mobile(s) d'agitation ou d'une colonne à bulles. Le temps de fermentation est habituellement supérieur à 30 heures, mais généralement compris entre 40 et 100 heures.

La productivité est mesurée en fonction de la quantité d'exopolysaccharide produit, exprimée en grammes, par rapport au kg de moût, par heure de fermentation. Avec le procédé de l'invention, une amélioration en productivité de 3 à 15 %, et de préférence de 5 à 10 % a été observée.

Après achèvement de la fermentation, l'exopolysaccharide peut être récupéré du moût et purifié selon les méthodes connues telles que filtration, concentration, cristallisation ou extraction par solvants.

L'invention couvre également les exopolysaccharides obtenus ou susceptibles d'être obtenus par le procédé. Elle couvre plus particulièrement la gomme xanthane produite par le procédé de l'invention.

La gomme xanthane obtenue selon le procédé de l'invention, en solution aqueuse à 1% dans l'eau distillée, présente une transparence élevée, c'est-à-dire de l'ordre de 70 à 95 % ou encore de l'ordre de 80 à 95%. La transmittance de la solution aqueuse est mesurée par spectrophotométrie à 600 nm.

Les exemples suivants illustrent la présente invention sans toutefois en limiter la portée .

**EXEMPLES**

**Exemple 1**

Cet exemple décrit les phases de préculture 1 et 2 pour le *Xanthomonas campestris.*

Préculture 1:

Composition du milieu de préculture :

- Extrait de levure    3 g/l    Oxoïd
- Extrait de malt    3 g/l    Oxoïd
- peptone de soja    5 g/l    Oxoïd
- Saccharose    10 g/l    Eurosucre
- pH ajusté à 7 par $H_2SO_4$
- qsp 1 litre avec de l'eau potable

Tous les constituants sont mis en solutions dans 1 litre d'eau potable, homogénéisés par agitation magnétique et répartis dans les erlenmeyers de 500 ml par fraction de 112 ml.
La préparation est autoclavée 30 minutes à 120°C.

La souche est initialement conservée sous la forme de tubes congelés à -196°C par le procédé de congélation dans les vapeurs d'azote liquide.

Pour une congélation azote liquide, on réalise une préculture sur un milieu spécifique ayant la composition suivante :

- extrait de malt    3 g    (procuré auprès de Oxoïd)
- extrait de levure    3 g    (Oxoïd)
- peptone de soja    5 g    (Oxoïd)
- glucose    10 g    (procuré auprès de Prolabo)
- eau de source qsp 1 l.

Pour la préparation du milieu, tous les ingrédients sont dispersés dans l'eau de source. Le pH est ajusté à 6,5 avec $H_2SO_4$ 10 %. Le milieu est stérilisé 20 minutes à 120°C, à l'autoclave.

Après 24 heures d'incubation à 28°C sur secoueur giratoire à 220 tr/min et amplitude = 50 mm, 10 % en volume de glycérol pur stérile sont ajoutés à la culture. La culture est ensuite répartie dans des cryotubes de contenances variant de 1 ml à 10 ml, de préférence de 2 ml à 4 ml.

Ces tubes sont conservés dans les vapeurs d'azote liquide.

La préculture 1 est ensemencée à l'aide d'un cryotube préalablement décongelé à l'air ambiant. La totalité ou 50 % du cryotube sont introduits de façon stérile dans les erlenmeyers de 500 ml, dont le milieu à été autodavé et donc stérilisé de la façon décrite ci-dessus.

Le milieu ainsi ensemencé est incubé 24 heures à 28°C sur un secoueur giratoire à 220 tr/mn et une amplitude de 50 mm.

Après 24 heures d'incubation nous obtenons une préculture dont le pH varie de 7 à 7,5, dont la viscosité est comprise entre 50 et 500 mPa.s et dont la population bactérienne en *Xanthomonas campestris* est supérieure à $10^{10}$/ml.

Préculture 2 :

La préculture 1 sert pour ensemencer la préculture 2.

Composition du milieu de préculture 2 :

- Saccharose    10 g/l    Eurosucre
- Farine de germe de caroube    4 g/l    Meyhall AG
- $Na_2HPO_4$    3 g/l    Europhos
- Eau potable ou eau adoucie    qsp 1 litre
- pH ajusté à l'acide sulfurique 10% pour avoir 6,5 avant stérilisation.

Tous les constituants sont mis en suspension dans 1 litre d'eau potable et le pH est austé à 6,5. Le milieu complet est autoclavé pendant 30 mn à 120°C après avoir réparti ce dit milieu dans des erlenmeyers de 500 ml par fraction de 112 ml.

Ces erlenmeyers sont ensuite ensemencés avec 0 ;1 à 0 ;2ml de la préculture 1. Ces erlenmeyers sont mis à incuber 24 à 30 heures heures à 28°C sur un secoueur giratoir à 220 tr/mn et une amplitude de 50 mm.

Après 24 à 30 heures d'incubation nous obtenons une préculture dont le pH varie de 5,8 à 7,1, dont la viscosité est comprise entre 100 et 1000 mPa.s et dont la population bactérienne en *Xanthomonas campestris* est supérieure à $10^9$ /ml

**Exemple 3**

Cet exemple décrit la préparation et l'obtention de l'exopolysaccharide selon deux procédés de fermentation, l'un avec une source d'azote organique et l'autre avec une source mixte d'azote organique et d'azote minérale.

Dans cet exemple deux étapes de "préculture" interviennent. Ces étapes ont lieu en erlenmeyers de 500 ml, ce qui correspond à 100 ml de milieu (voir les exemples 1 et 2).

L'étape de production qui correspond à l'étape au cours de laquelle la souche bactérienne produit le polysaccharide, a lieu en fermenteur de 20 litres, dont 15 litres utiles.

Etape préculture 1 et 2:

Les étapes de préculture 1 et 2 sont effectuées de la même manière que dans les exemples 1 et 2.

Etape de production:

Milieu 1:

La dernière étape est l'étape de production de l'exopolysaccharide.
Le milieu du fermenteur 1 a la composition suivante :

- ☐ saccharose         42 g         Eurosucre
- ☐ Farine de germe de caroube         6 g         Meyhall AG
- ☐ $MgSO_4,7H_2O$       0,25 g         Bittersalz
- ☐ $Na_2HPO_4$       2 g       Prolabo
- ☐ antimousse organique         0,5 ml
- ☐ eau potable ou adoucie qsp 1l

La préparation des sources azotées et de carbohydrates est réalisée séparément.
*Saccharose* ⇆ Qsp grammes de glucose sont dissous dans qsp 3 l d'eau adoucie ou potable dans un flacon deMariotte. Le pH est abaissé à 5,2 par $H_2SO_4$ 10 %. La solution est stérilisée en flacon de Mariotte 45 minutes à 120°C à l'autoclave.
Farine de germe de caroube + sels ⇆ Qsp grammes de farine de germe de caroube, 30 g de $Na_2HPO_4$, 3,75 g de $MgSO_4,7H_2O$, et 7,5 ml d'antimousse sont dissous dans qsp 7 l d'eau adoucie. Le pH est ajusté à 6 avec $H_2SO_4$ 10 %. Ce mélange est stérilisé *in situ* 45 minutes à 120°C.
Soude 1N ⇆ 40 g de pastilles de NaOH sont dissous dans qsp 1l d'eau distillée. La solution est stérilisée en flacon de Mariotte 30 minutes à 120°C à l'autoclave.
Quand tous les ingrédients sont à 28°C, ils sont mélangés dans le fermenteur. Le fermenteur est ensuite inoculé avec qsp de préculture 2.
Les conditions de fermentation dans le fermenteur sont les suivantes :

Agitation ⇆ 200 tr/min de 0 à 20 heures d'âge, puis 400 tr/min. jusqu'à la fin de la fermentation
Aération ⇆ 400 l/h de 0 à 18 heures puis 825 l/h de 24 heures jusqu'à la fin de la fermentation
La température est régulée à 28°C.
Le pH est régulé à 6,8 par NaOH 1N.
La pression est la pression atmosphérique .

Milieu 2:

Le milieu 2, qui peut être une alternative au milieu 1, a la composition suivante :

- ☐ Saccharose         42 g/l         (Eurosucre)
- ☐ $NH_4NO_3$       1,15 g/l       (Atochem)
- ☐ $MgSO_4,7H_2O$       0,25 g/l       (Bitter salz)
- ☐ $(NH4)_2HPO_4$       0,217 g/l       (Europhos)
- ☐ Soluble de farine de germe de caroube         36 g/l         (Meyhall AG)
- ☐ antimousse organique         0,2 ml
- ☐ eau adoucie qsp 1 l

Saccharose ⇆ Qsp grammes de glucose sont dissous dans qsp 3 l d'eau adoucie. Le pH est ajusté à 5 par $H_2SO_4$ 10 %. La solution est stérilisée en flacon de Mariotte 30 minutes à 120°C à l'autoclave.
Azote + sels ⇆ 17.25 g de $NH_4NO_3$, 3.75 g de $MgSO_4$, $7H_2O$, 3.22 gr de $(NH_4)2HPO_4$, 525 gr de soluble de farine de germe de caroube et 3 ml d'antimousse sont dissous dans qsp 7l d'eau adoucie. Le pH de cette solution est ajusté à 6 avec $H_2SO_4$ 10 %. Ce mélange est stérilisé *in situ* 45 minutes à 120°C.
Soude 1N ⇆ 40 g de pastilles de NaOH sont dissous dans qsp 1l d'eau distillée. La solution est stérilisée en flacon de Mariotte 30 minutes à 120°C à l'autoclave.
Le soluble de farine de germe de caroube est préparé par dillution de farine à 6 à 15% dans de l'eau adoucie. Cette solution peut ou non être traitée par des enzymes type protéases alcalines, acides et/ou neutres; des lipases ; des phytases; des phosphatases alcalines, acides et/ou neutres ; des amylases, avant d'être décantée ou non sur un décanteur rotatif horizontal afin d'éliminer les impuretés pouvant nuire à la qualité du produit final.
Quand tous les ingrédients sont à 28°C, ils sont mélangés dans le fermenteur (milieu 1 ou 2). Le fermenteur est ensuite inoculé avec qsp de préculture 2.
Les conditions de fermentation dans le fermenteur 2 sont les suivantes :

Agitation ⇆ 200 tr/min de 0 à 20 heures d'âge, puis 400 tr/min jusqu'à la fin de la fermentation
Aération ⇆ 400 l/h de 0 à 24 heures puis 825 l/h de 24 heures jusqu'à la fin de la fermentation
La température est régulée à 28°C.
Le pH est régulé à 6,8 par NaOH 1N.
La pression est la pression atmosphérique ou sous une pression pouvant aller de 0,5 à 4 bars.

Résultats de fermentation:

Selon le milieu de culture étudié, les durées de fermentations varient de 45 à 65 heures, les matières sèches précipitables à l'isopropanol varient de 20 à 30 g/kg, et le rendement pondéral par rapport à la source de carbone mise en oeuvre varie de 50 à 70 %. Le moût de fermentation obtenu présente une luminosité et une brillance jamais observées avec n'importe quelle autre source d'azote.

**Revendications**

**1.** Procédé de production d'exopolysaccharides par fermentation de microorganismes **caractérisé en ce que** l'on conduit la fermentation dans un milieu nutritif comportant au moins une source de carbone assimilable par les microorganismes et au moins une source organique d'azote, ladite source dérivant d'une fraction de la graine de caroube, de guar, de cassia ou de tara.

2. Procédé selon la revendication 1, **caractérisé en ce que** la source organique d'azote dérive de la fraction de la graine de caroube.

3. Procédé selon la revendication 1 ou 2, **caractérisé en ce que** la fraction de la graine de caroube possède une teneur en protéines d'au moins 45 %, de préférence d'au moins 50 %, et plus préférentiellement d'au moins 60 %, en poids par rapport au poids sec de la matière sèche.

4. Procédé selon la revendication 3, **caractérisé en ce que** les protéines ont avantageusement un taux élevé en arginine, en glutamine et/ou acide glutamique, et en lysine.

5. Procédé selon l'une quelconque des revendications 1 à 4, **caractérisée en ce que** la fraction de la graine de caroube contient une teneur en lipides d'au moins 4 %, avantageusement d'au moins 5 %, et encore plus avantageusement varie entre 7 et 15% en poids en poids par rapport à la matière sèche.

6. Procédé selon l'une quelconque des revendications 1 à 5, **caractérisé en ce que** la fraction est le germe de graine de caroube.

7. Procédé selon l'une quelconque des revendications 1 à 6, **caractérisé en ce que** la fraction de la graine de caroube est sous forme d'une farine.

8. Procédé selon la revendication 7, **caractérisé en ce que** la granulométrie de la farine fluctue entre 10 et 150 microns.

9. Procédé selon l'une quelconque des revendications 1 à 8, **caractérisé en ce que** l'on conduit la fermentation dans un milieu nutritif comportant au moins une source minérale d'azote.

10. Procédé selon la revendication 9, **caractérisé en ce que** la source d'azote minérale est choisie parmi les nitrates d'ammonium ou de sodium, les phosphates ou les sulfates d'ammonium, sulfate de magnésium, sulfate de potassium ou de sodium, seuls ou en mélange.

11. Procédé selon l'une quelconque des revendications 1 à 10, **caractérisé en ce que** la concentration en source organiques et éventuellement minérales d'azote dans le milieu de fermentation est comprise entre 1 et 80 g/l, de préférence entre 3 et 50 g/l, et plus préférentiellement entre 5 et 30 g/l.

12. Procédé selon l'une quelconque des revendications 1 à 10, **caractérisé en ce que** la source de carbone assimilable est choisie parmi le glucose ou le saccharose.

13. Procédé selon l'une quelconque des revendications 1 à 12, **caractérisé en ce que** la concentration en source de carbone assimilable est comprise entre 1 et 100 g/l, et de préférence entre 15 et 80 g/.

14. Procédé selon l'une quelconque des revendications 1 à 13, **caractérisé en** l'on conduit la fermentation des microorganismes en l'absence d'enzyme.

15. Procédé selon l'une quelconque des revendications 1 à 14, **caractérisé en ce que** l'on conduit la fermentation à une température comprise entre 15 et 100°C, de préférence entre 25 et 80°C, et plus particulièrement entre 25 et 35°C.

16. Procédé selon l'une quelconque des revendications 1 à 15, **caractérisé en ce que** le microorganisme est choisi dans le groupe des bactéries du genre *Xanthomonas,* du genre *Alcaligenes,* du genre *Agrobacterium,* du genre *Arthrobacter,* du genre *Azotobacter,* du genre *Pseudomonas,* du genre *Corynebacterium,* des champignons du genre *Sclerotium*, du genre *Aspergillus,* et des levures du genre *Hansenula.*

17. Exopolysaccharide obtenu par un procédé selon l'une quelconque des revendications 1 à 16.

**Patentansprüche**

1. Verfahren zur Herstellung von Exopolysacchariden durch Fermentation von Mikroorganismen, **dadurch gekennzeichnet, dass** die Fermentation in einem Nährmedium durchgeführt wird, das wenigens eine durch die Mikroorganismen assimilierbare Kohlenstoffquelle und wenigstens eine organische Stickstoffquelle umfasst, wobei diese Quelle von einer Fraktion von dem Johannisbrotsamen, Guarkern, Johannisbeerensamen oder Tara-Samen abstammt.

2. Verfahren gemäß Anspruch 1, **dadurch gekennzeichnet, dass** die organische Stickstoffquelle von der Fraktion von dem Johannisbrotsamen abstammt.

3. Verfahren gemäß Anspruch 1 oder 2, **dadurch gekennzeichnet, dass** die Fraktion von dem Johannisbrotsamen einen Proteingehalt von wenigstens 45 Gew.-%, bevorzugt wenigstens 50 Gew.-%, und besonders bevorzugt wenigstens 60 Gew.-%, bezogen auf das Trockengewicht des Trockenmaterials, besitzt.

**4.** Verfahren gemäß Anspruch 3, **dadurch gekennzeichnet, dass** die Proteine bevorzugt einen erhöhten Prozentsatz an Arginin, an Glutamin und/oder Glutaminsäure und an Lysin aufweisen.

**5.** Verfahren gemäß einem der Ansprüche 1 bis 4, **dadurch gekennzeichnet, dass** die Fraktion von dem Johannisbrotsamen einen Lipidgehalt von wenigstens 4 Gew.-%, vorteilhafterweise von wenigstens 5 Gew.-% enthält, und noch vorteilhafterweise einen, der zwischen 7 und 15 Gew.-% variiert, bezogen auf das Trockenmaterial.

**6.** Verfahren gemäß einem der Ansprüche 1 bis 5, **dadurch gekennzeichnet, dass** die Fraktion der Keim von Johannisbrotsamen ist.

**7.** Verfahren gemäß einem der Ansprüche 1 bis 6, **dadurch gekennzeichnet, dass** die Fraktion von dem Johannisbrotsamen in Form von einem Mehl vorliegt.

**8.** Verfahren gemäß Anspruch 7, **dadurch gekennzeichnet, dass** die Granulometrie des Mehls zwischen 10 und 150 Mikrometer schwankt.

**9.** Verfahren gemäß einem der Ansprüche 1 bis 8, **dadurch gekennzeichnet, dass** die Fermentation in einem Nährmedium durchgeführt wird, das wenigstens eine anorganische Stickstoffquelle umfasst.

**10.** Verfahren gemäß Anspruch 9, **dadurch gekennzeichnet, dass** die anorganische Stickstoffquelle aus den Nitraten von Ammonium oder Natrium, den Phosphaten oder den Sulfaten von Ammonium, Magnesiumsulfat, Kaliumsulfat oder Natriumsulfat, einzeln oder als Mischung, ausgewählt ist.

**11.** Verfahren gemäß einem der Ansprüche 1 bis 10, **dadurch gekennzeichnet, dass** die Konzentration an organischen und gegebenenfalls anorganischen Stickstoffquellen im Fermentationsmedium zwischen 1 und 80 g/l liegt, bevorzugt zwischen 3 und 50 g/l, und noch bevorzugter zwischen 5 und 30 g/l.

**12.** Verfahren gemäß einem der Ansprüche 1 bis 10, **dadurch gekennzeichnet, dass** die assimilierbare Kohlenstoffquelle aus der Glukose und der Saccharose ausgewählt ist.

**13.** Verfahren gemäß einem der Ansprüche 1 bis 12, **dadurch gekennzeichnet, dass** die Konzentration an assimilierbarer Kohlenstoffquelle zwischen 1 und 100 g/l, und bevorzugt zwischen 15 und 80 g/l liegt.

**14.** Verfahren gemäß einem der Ansprüche 1 bis 13, **dadurch gekennzeichnet, dass** die Fermentation der Mikroorganismen in Abwesenheit von Enzymen durchgeführt wird.

**15.** Verfahren gemäß einem der Ansprüche 1 bis 14, **dadurch gekennzeichnet, dass** die Fermentation bei einer Temperatur durchgeführt wird, die zwischen 15 und 100°C, bevorzugt zwischen 25 und 80°C, und insbesondere zwischen 25 und 35°C liegt.

**16.** Verfahren gemäß einem der Ansprüche 1 bis 15, **dadurch gekennzeichnet, dass** der Mikroorganismus aus der Gruppe der Bakterien der Gattung *Xanthomonas,* der Gattung *Alcaligenes,* der Gattung *Agrobacterium,* der Gattung *Arthrobacter,* der Gattung *Azotobacter,* der Gattung *Pseudomonas,* der Gattung *Corynebacterium,* der Pilze der Gattung *Sclerotium,* der Gattung *Aspergillus* und der Hefen der Gattung *Hansenula* ausgewählt wird.

**17.** Exopolysaccharid, erhalten nach einem Verfahren gemäß einem der Ansprüche 1 bis 16.

**Claims**

**1.** Process for the production of exopolysaccharides by fermentation of microorganisms **characterized in that** the fermentation is carried out in a nutrient medium containing at least one carbon source which can be assimilated by the microorganisms and at least one organic source of nitrogen, said source deriving from a fraction of the carob, guar, blackcurrant or tara seed.

**2.** Process according to claim 1, **characterized in that** the organic source of nitrogen is derived from the fraction of the carob seed.

**3.** Process according to claim 1 or 2, **characterized in that** the fraction of the carob seed has a protein content of at least 45%, preferably at least 50%, and more preferably of at least 60% by weight relative to the dry weight of the dry matter.

**4.** Process according to claim 3, **characterized in that** the proteins advantageously have a high arginine, glutamine and/or glutamic acid, and lysine content.

**5.** Process according to any one of claims 1 to 4, **characterized in that** the fraction of the carob seed has a lipids content of at least 4 %, advantageously at least 5 %, and even more advantageously one that varies between 7 and 15 % by weight relative to the dry matter.

6. Process according to any one of claims 1 to 5, **characterized in that** the fraction is the carob seed germ.

7. Process according to any one of claims 1 to 6, **characterized in that** the fraction of the carob seed is in the form of a flour.

8. Process according to claim 7, **characterized in that** the grain size of the flour fluctuates between 10 and 150 microns.

9. Process according to any one of claims 1 to 8, **characterized in that** the fermentation is carried out in a nutrient medium containing at least one mineral source of nitrogen.

10. Process according to claim 9, **characterized in that** the mineral source of nitrogen is chosen from the ammonium or sodium nitrates, the ammonium phosphates or sulphates, magnesium sulphate, potassium or sodium sulphate, alone or mixed.

11. Process according to any one of claims 1 to 10, **characterized in that** the concentration of organic sources and where appropriate nitrogen mineral sources in the fermentation medium is between 1 and 80 g/l, preferably between 3 and 50 g/l, and more preferably between 5 and 30 g/l.

12. Process according to any one of claims 1 to 10, **characterized in that** the assimilable carbon source is chosen from glucose or sucrose.

13. Process according to any one of claims 1 to 12, **characterized in that** the concentration of the assimilable carbon source is between 1 and 100 g/l, and preferably between 15 and 80 g/l.

14. Process according to any one of claims 1 to 13, **characterized in that** the fermentation of the microorganisms is carried out in the absence of enzymes.

15. Process according to any one of claims 1 to 14, **characterized in that** the fermentation is carried out at a temperature of between 15 and 100°C, preferably between 25 and 80°C, and more particularly between 25 and 35°C.

16. Process according to one of claims 1 to 15, **characterized in that** the microorganism is chosen from the group of the bacteria of the genus *Xanthomonas,* of the genus *Alcaligenes,* of the genus *Agrobacterium,* of the genus *Arthrobacter,* of the genus *Azotobacter,* of the genus *Pseudomonas,* of the genus *Corynebacterium,* of the fungi of the genus *Sclerotium,* of the genus *Aspergillus,* and the yeasts of the genus *Hansenula.*

17. Exopolysaccharide obtained by a process according to any one of claims 1 to 16.